# EUROPEAN PATENT APPLICATION

(11) **EP 2 238 970 A1**
(43) Date of publication of application: **13.10.2010**
(21) Application number: 08867516.0
(22) Date of filing: 19.12.2008
(51) Int. Cl.: A61K 8/44, A61K 8/06, A61K 31/221, A61P 3/04, A61P 17/00, A61P 17/08, A61P 17/14, A61Q 19/00

(54) **EMULSION PREPARATION FOR EXTERNAL APPLICATION TO SKIN AND COSMETIC PREPARATION**

(30) Priority: 28.12.2007 JP 2007339828
(71) Applicant: Showa Denko K.K., Tokyo 105-8518 (JP)
(72) Inventor: AOKI, Hirobumi, Chiba-shi Chiba 267-0056 (JP); KAMACHI, Harumi, Chiba-shi Chiba 267-0056 (JP)
(74) Representative: Strehl Schübel-Hopf & Partner
(86) International application number: PCT/JP2008/073163
(87) International publication number: WO 2009/084471

(57) **Abstract**

An object of the present invention is to provide emulsified skin external preparations and cosmetics which contain a branched acyl carnitine and have excellent emulsion stability. An emulsified skin external preparation according to the present invention includes 0.01 to 10% by mass of a carnitine derivative represented by the following Formula (1) and/or a carnitine derivative salt represented by the following Formula (2), and 0.01 to 20% by mass of a nonionic surfactant. 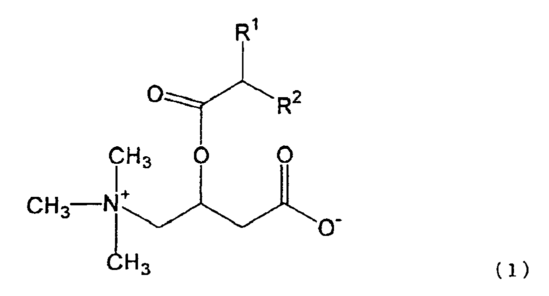 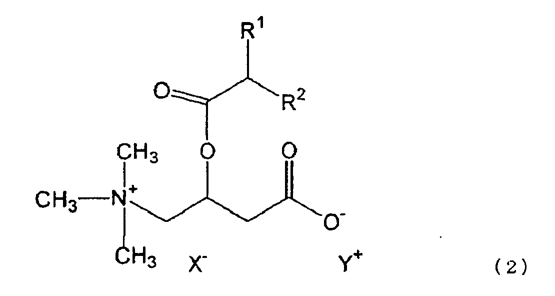 In Formula (1), R¹ and R² are each independently a C1-18 optionally branched, saturated or unsaturated aliphatic hydrocarbon group. In Formula (2), R¹ and R² are the same as in Formula (1), X⁻ is a specific anion and Y⁺ is a specific cation.

## Description

### FIELD OF THE INVENTION

The present invention relates to emulsified skin external preparations and cosmetics. In more detail, the present invention relates to stable emulsified skin external preparations and cosmetics that contain carnitine derivatives and/or salts thereof and specific surfactants.

### BACKGROUND OF THE INVENTION

Carnitine is well-known to play an important role in human lipid metabolism. In cells, the carnitine is enzymatically bound to the fatty acids from fats and transports the fatty acids into the organelle mitochondria where the fat is burned, serving as a carrier.

On the other hand, excessive lipids lead to obesity as a result of the accumulation of subcutaneous fat, and cause many cosmetic and QOL (quality of life) problems, including cellulite formation, shiny and greasy skin due to excessive sebum, seborrheic dermatitis and consequent hair loss, acnes, body odor, and skin aging due to decreased lipid metabolism.

The lipid metabolism requires that the fatty acids be transported into the mitochondria, and the carnitine is essential to the transportation. Accordingly, the lipid metabolic rate is dependent on the amount of carnitine in the cells. Increasing the carnitine concentration in target tissues for lipid metabolism will stimulate the lipid metabolism and will prevent and eliminate excessive lipids and problems associated therewith.

Many studies have then focused on stimulating the lipid metabolism by percutaneous absorption of carnitine derivatives formulated in skin external preparations. For example, Patent Document 1 discloses a slimming skin external preparation containing palmitoyl-L-carnitine (L-carnitine palmitate). Patent Document 2 discloses a carnitine fatty acid ester having a branched acyl group, and this carnitine derivative has improved stability in formulations.
Patent Document 1: French Patent No. 2694195 specification
Patent Document 2: JP-A-2007-119441 DISCLOSURE OF THE INVENTION

### PROBLEMS TO BE SOLVED BY THE INVENTION

However, the branched acyl carnitine is an amphoteric substance having a carboxyl group and a quaternary ammonium group in the molecule, and is dissociated in solutions at a wide range of pH values to function as an electrolyte. Electrolytes are well-known to inhibit the emulsification with surfactants. In particular, the branched acyl carnitine destabilizes the emulsification with aqueous media or the like in so-called emulsified formulations such as creams, milky lotions and emulsions which are widely employed in skin external preparations or cosmetics. Accordingly, in spite of its usefulness, it has been difficult to formulate the branched acyl carnitine in high concentrations in emulsified formulations.

The present invention is aimed at solving the problems as described above. It is therefore an object of the present invention to provide emulsified skin external preparations and cosmetics which comprise branched acyl carnitines and show excellent emulsion stability.

### MEANS FOR SOLVING THE PROBLEMS

The present inventors studied diligently and have found that emulsified skin external preparations and cosmetics which comprise branched acyl carnitines while showing excellent emulsion stability can be easily produced with use of nonionic surfactants, in particular polyoxyethylene polyhydric alcohol fatty acid esters. It has been further found that the long-term stability of the formulations is improved by, in addition to the combination, adjusting the pH of the emulsified skin external preparations to a specific range. The present invention has been completed based on the findings. In detail, the present invention is concerned with the following [1] to [12].

[1] An emulsified skin external preparation comprising 0.01 to 10% by mass of a carnitine derivative represented by the following Formula (1) and/or a carnitine derivative salt represented by the following Formula (2), and 0.01 to 20% by mass of a nonionic surfactant;

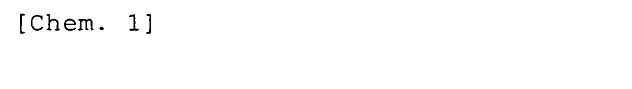

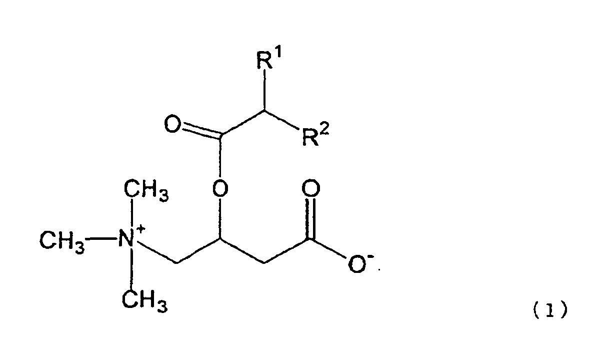

wherein R¹ and R² are each independently a C1-18 optionally branched, saturated or unsaturated aliphatic hydrocarbon group;

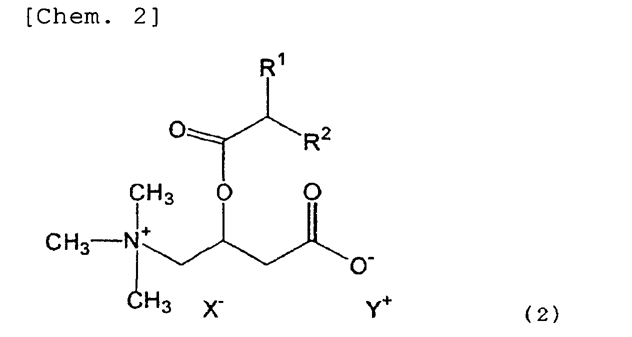

wherein R¹ and R² are the same as R¹ and R² in Formula (1), X⁻ is an inorganic or organic anion providing electrical neutrality with the cation part of the carnitine derivative, and Y⁺ is an inorganic or organic cation providing electrical neutrality with the anion part of the carnitine derivative.

[2] The emulsified skin external preparation as described in [1] above, wherein R¹ and R² in Formulae (1) and (2) are each independently a C3-16 optionally branched, saturated or unsaturated aliphatic hydrocarbon group.

[3] The emulsified skin external preparation as described in [1] above, wherein either of R¹ and R² in Formulae (1) and (2) is a C6 linear alkyl group and the other is a C8 linear alkyl group.

[4] The emulsified skin external preparation as described in any one of [1] to [3] above, wherein X⁻ in Formula (2) is selected from the group consisting of hydroxide ion, nitrate ion, sulfate ion, carbonate ion, hydrogen carbonate ion, halide ion, formate ion, acetate ion, citrate ion, tartrate ion, oxalate ion, fumarate ion, C3-20 optionally branched, saturated or unsaturated fatty acid anion, carnitine anion, carnitine derivative anion, ascorbate anion, ascorbylphosphate anion and ascorbylphosphate derivative anion.

[5] The emulsified skin external preparation as described in any one of [1] to [4] above, wherein Y⁺ in Formula (2) is selected from the group consisting of hydrogen ion, sodium ion, potassium ion, calcium ion, magnesium ion, zinc ion, ammonium ion, carnitine cation and carnitine derivative cation.

[6] The emulsified skin external preparation as described in any one of [1] to [5] above, wherein the nonionic surfactant has a polyoxyethylene skeleton.
[7] The emulsified skin external preparation as described in [6] above, wherein the nonionic surfactant is a nonionic ether ester surfactant.

[8] The emulsified skin external preparation as described in [7] above, wherein the nonionic surfactant is a polyoxyethylene polyhydric alcohol fatty acid ester.
[9] The emulsified skin external preparation described in any one of [1] to [8] above, wherein the nonionic surfactant is a polyoxyethylene sorbitan fatty acid ester.

[10] The skin external preparation as described in any one of [1] to [9] above, wherein the preparation has a pH in the range of 5.0 to 9.5.
[11] The skin external preparation as described in any one of [1] to [9] above, wherein the emulsion has a pH in the range of 5.5 to 8.5.

[12] The emulsified skin external preparation as described in any one of [1] to [11] above, which is used as a cosmetic.

### ADVANTAGEOUS EFFECTS OF THE INVENTION

The emulsified skin external preparations and cosmetics of the present invention achieve high emulsion stability in spite of the inclusion of the branched acyl carnitines which are destructive to emulsified formulations. In particular, the formulations have an excellent touch and small changes in appearance even after long-term storage, being used in cosmetics in which the touch or appearance greatly influences the commercial values thereof.

Dermatological preparations are typically used in small amounts taken out of the container. Maintaining the high homogeneity of the components in the formulations leads to changeless storage and provision without temporal changes of the components in each use of the formulations. The emulsified skin external preparations of the present invention can maintain quality during storage and ensure designed effects of the formulations over a long term after opening till the formulation is finished. The emulsified skin external preparations of the present invention are formulations which can stably provide the effects of the branched acyl carnitine as well as the effects of other components formulated therewith.

### PREFERRED EMBODIMENTS FOR CARRYING OUT THE INVENTION

The present invention will be described in detail below.
An emulsified skin external preparation according to the present invention contains a specific carnitine derivative and/or a salt thereof. In detail, the emulsified skin external preparation of the present invention contains a specific carnitine derivative or a specific carnitine derivative salt, or contains both a specific carnitine derivative and a salt thereof.

### (Carnitine derivatives)

The carnitine derivatives used in the emulsified skin external preparations of the present invention are compounds represented by Formula (1) below:

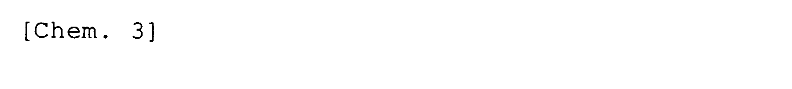

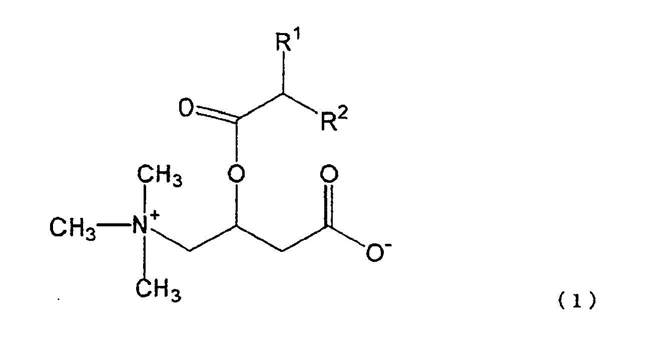

In Formula (1), R¹ and R² are each independently a C1-18 optionally branched, saturated or unsaturated aliphatic hydrocarbon group. In a preferred embodiment, either of R¹ and R² is a C1-16 optionally branched, saturated or unsaturated aliphatic hydrocarbon group and the other is a C3-16 optionally branched, saturated or unsaturated aliphatic hydrocarbon group. In a more preferred embodiment, R¹ and R² are each independently a C3-16 optionally branched, saturated or unsaturated aliphatic hydrocarbon group. In a still more preferred embodiment, they are each independently a C4-12 optionally branched, saturated or unsaturated aliphatic hydrocarbon group.

The carnitine part in the carnitine derivatives is generally L-form. The carnitine derivatives contain an α-branched acyl group having R¹ and R². When R¹ and R² are different from each other, the α-carbon atom at the branching point is an asymmetric carbon atom, and optical isomers are possible. The carnitine derivatives used in the emulsified skin external preparations of the present invention are not particularly limited, and any optical isomers and mixtures thereof may be used.

The carnitine derivatives contain an α-branched acyl group having R¹ and R², and the acyl group is hardly hydrolyzed in the presence of an aqueous medium because the electron donation to the ester bond is reduced by the branched chain. Hence, it is thought that the derivative can stably exist for a long-term.

Examples of the saturated aliphatic hydrocarbon groups include linear or branched alkyl groups such as methyl group, ethyl group, n-propyl group, isopropyl group, n-butyl group, isobutyl group, sec-butyl group, tert-butyl group, n-pentyl group, n-hexyl group, 2-methylpentyl group, 3-methylpentyl group, 4-methylpentyl group, 2-ethylbutyl group, n-heptyl group, 2-methylhexyl group, 3-methylhexyl group, 4-methylhexyl group, 2-ethylpentyl group, 3-ethylpentyl group, n-octyl group, 2-methylheptyl group, 3-methylheptyl group, 4-methylheptyl group, 5-methylheptyl group, 6-methylheptyl group, 2-ethylhexyl group, 3-ethylhexyl group, 4-ethylhexyl group, 2-propylpentyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group and isostearyl group.

Examples of the unsaturated aliphatic hydrocarbon groups include linear or branched alkenyl groups such as 10-undecenyl group, 9-hexadecenyl group, cis-9-octadecenyl group, 11-octadecenyl group, cis,cis-9,12-octadecadienyl group, 9,12,15-octadecatrienyl group, 6,9,12-octadecatrienyl group and 9,11,13-octadecatrienyl group.

Preferred combinations of R¹ and R², namely R¹/R², include methyl group/methyl group, methyl group/ethyl group, methyl group/n-propyl group, methyl group/isopropyl group, methyl group/n-butyl group, methyl group/n-pentyl group, methyl group/n-hexyl group, methyl group/n-octyl group, methyl group/n-decyl group, methyl group/n-tetradecyl group, methyl group/n-hexadecyl group, ethyl group /ethyl group, ethyl group/n-propyl group, ethyl group/isopropyl group, ethyl group/n-butyl group, ethyl group/isopropyl group, ethyl group/n-butyl group, ethyl group/n-pentyl group, ethyl group/n-hexyl group, ethyl group/n-octyl group, ethyl group/n-decyl group, ethyl group/n-tetradecyl group, ethyl group/n-hexadecyl group, n-propyl group/n-propyl group, n-propyl group/n-butyl group, n-propyl group/n-pentyl group, n-propyl group/n-hexyl group, n-butyl group/n-hexyl group, n-butyl group/n-octyl group and n-hexyl group/n-octyl group. A particularly preferred combination is n-hexyl group/n-octyl group.

The emulsified skin external preparations of the present invention may contain one, or two or more carnitine derivatives specified by the above combination of R¹ and R².

### (Carnitine derivative salts)

The salts of carnitine derivatives used in the emulsified skin external preparations of the present invention are compounds represented by Formula (2) below:

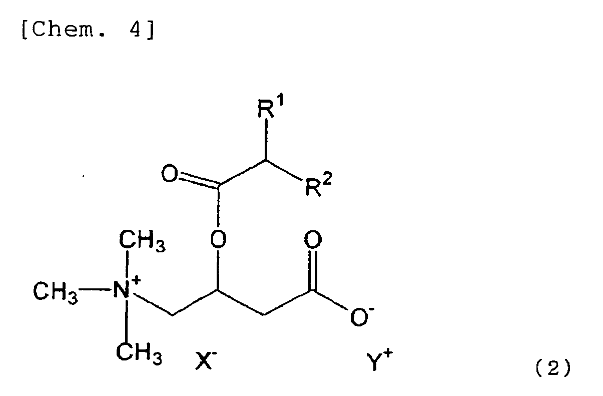

In Formula (2), X⁻ is an inorganic or organic anion providing electrical neutrality with the cation part of the carnitine derivative, and is preferably a pharmaceutically acceptable anion.
Specific examples thereof include inorganic ions such as hydroxide ion, nitrate ion, sulfate ion, carbonate ion, hydrogen carbonate ion and halide ion; and
organic ions such as formate ion, acetate ion, citrate ion, tartrate ion, oxalate ion, fumarate ion, C3-20 optionally branched, saturated or unsaturated fatty acid anion, carnitine anion, carnitine derivative anion, ascorbate anion, ascorbylphosphate anion and ascorbylphosphate derivative anion.

Of these, the hydroxide ion, halide ion, citrate ion, carnitine anion and carnitine derivative anion are preferable from the viewpoint of formulating properties of the skin external preparations, in particular cosmetics.

In Formula (2), Y⁺ is an inorganic or organic cation providing electrical neutrality with the anion part of the carnitine derivative, and is preferably a pharmaceutically acceptable cation.
Specific examples thereof include hydrogen ion, sodium ion, potassium ion, calcium ion, magnesium ion, zinc ion, ammonium ion, carnitine cation and carnitine derivative cation.

Of these, the hydrogen ion, sodium ion, potassium ion, carnitine cation and carnitine derivative cation are preferable from the viewpoint of formulating properties of the skin external preparations, in particular cosmetics.
In Formula (2), R¹ and R² are the same as defined in Formula (1).

The emulsified skin external preparations of the present invention may contain one, or two or more carnitine derivative salts specified by the above combination of X⁻, Y⁺, R¹ and R².

### (Nonionic surfactants)

The emulsified skin external preparations contain a nonionic surfactant together with the carnitine derivative and/or the salt thereof.

Examples of the nonionic surfactants for use in the present invention include:
polyoxyethylene alkyl ethers,
polyoxyethylene alkylphenyl ethers,
polyoxyethylene polyoxypropylene alkyl ethers,
polyoxyethylene fatty acids,
polyoxyethylene fatty acid glycerides,
polyoxyethylene sorbitan fatty acid esters,
polyoxyethylene castor oils,
polyoxyethylene hydrogenated castor oils,
polyoxyethylene castor oil fatty acid esters,
polyoxyethylene hydrogenated castor oil fatty acid esters,
polyoxyethylene sorbitol fatty acid esters,
glycerol esters of fatty acids,
polyglycerol esters of fatty acids,
sorbitan fatty acid esters,
sucrose fatty acid esters,
alkyl glucosides,
alkyl polyglucosides and
N-alkyl dimethyl amine oxides.

Among these, from the viewpoint of emulsion stability, nonionic surfactants having a polyoxyethylene skeleton are preferable, and nonionic ether ester surfactants having a polyoxyethylene skeleton are particularly preferable.

Examples of the nonionic surfactants having a polyoxyethylene skeleton include:
polyoxyethylene alkyl ethers,
polyoxyethylene alkylphenyl ethers,
polyoxyethylene polyoxypropylene alkyl ethers,
polyoxyethylene fatty acids,
polyoxyethylene fatty acid glycerides,
polyoxyethylene sorbitan fatty acid esters,
polyoxyethylene castor oils,
polyoxyethylene hydrogenated castor oils,
polyoxyethylene castor oil fatty acid esters,
polyoxyethylene hydrogenated castor oil fatty acid esters and
polyoxyethylene sorbitol fatty acid esters.

Of these, the nonionic ether ester surfactants having a polyoxyethylene skeleton are:
polyoxyethylene fatty acids,
polyoxyethylene fatty acid glycerides,
polyoxyethylene sorbitan fatty acid esters,
polyoxyethylene castor oils,
polyoxyethylene hydrogenated castor oils,
polyoxyethylene castor oil fatty acid esters,
polyoxyethylene hydrogenated castor oil fatty acid esters and
polyoxyethylene sorbitol fatty acid esters.

From the viewpoint of emulsion stability, polyoxyethylene polyhydric alcohol fatty acid esters are more preferably used, with examples including:
polyoxyethylene sorbitan fatty acid esters,
polyoxyethylene castor oil fatty acid esters,
polyoxyethylene hydrogenated castor oil fatty acid esters and
polyoxyethylene sorbitol fatty acid esters.

The nonionic surfactants may be used singly, or two or more kinds may be used in combination.

### (Emulsified skin external preparations and cosmetics)

The emulsified skin external preparations of the present invention contain the aforesaid carnitine derivatives and/or salts thereof, and the nonionic surfactants in specific amounts as will be described later. The carnitine derivatives and the salts thereof are effective in improving the lipid metabolism, and may be used for the purpose of improving and preventing skin disorders, aging and obesity associated with excessive lipids or decreased lipid metabolism. The emulsified skin external preparations of the present invention may be used as cosmetics, in particular lipid metabolism-improving cosmetics. The emulsified skin external preparations of the present invention used as cosmetics will be simply referred to as the cosmetics of the present invention hereinafter.

Examples of the purposes include slimming, cellulite prevention, skin tightening, prevention of shiny and greasy skin and smeared makeup due to excessive sebum, improvement and prevention of seborrheic dermatitis and consequent hair loss, acne prevention, body odor prevention, and anti skin aging, skin activation and skin caring by promoting the conversion of lipids into energy.

The fields of application of the cosmetics according to the present invention are not limited to those described above. The cosmetics may be used to add the effects of the carnitine derivatives and the salts thereof, to cosmetics having various effects and efficacies.

The emulsified skin external preparations of the present invention may contain the carnitine derivatives and/or the salts thereof at concentrations corresponding to the desired effects of the carnitine derivatives and/or the salts thereof. From the viewpoint of formulation stability, the concentration thereof is in the range of 0.01 to 10% by mass, preferably 0.05 to 5% by mass, and more preferably 0.05 to 5% by mass based on the total mass of the emulsified skin external preparation. The concentration represents the amount of the carnitine derivative when the carnitine derivative is used alone, or the amount of the carnitine derivative salt when the carnitine derivative salt is used alone, or the total amount of the carnitine derivative and the salt thereof when they are used in combination.

The emulsified skin external preparations of the present invention contain the nonionic surfactants in an amount of 0.01 to 20% by mass, preferably 0.05 to 10% by mass, and more preferably 0.05 to 5% by mass based on the total mass of the emulsified skin external preparation. The nonionic surfactants contained in this amount, in combination with the carnitine derivatives and/or the salts thereof, ensure that the emulsified formulations are formed very easily and stably and maintain good conditions and commercial values.

To maintain the formulation stability, the pH of the emulsified skin external preparations of the present invention is preferably adjusted in the range of 5.0 to 9.5. In particular, the emulsified skin external preparations achieve higher formulation stability and maintain good conditions and commercial values at a pH in the range of 5.5 to 8.5.

The emulsified skin external preparations and the cosmetics of the present invention may contain other components commonly used in general skin external preparations and cosmetics in addition to the carnitine derivatives and/or the salts thereof selected in a way as aforesaid and the nonionic surfactants selected in a way as aforesaid, while still achieving the effects of the invention.

Such components include:
hydrocarbons such as ozokerite, α-olefin oligomers, light isoparaffin, light liquid isoparaffin, squalene, squalane, synthetic squalane, vegetable squalane, ceresin, paraffin, polyethylene powder, polybutene, microcrystalline wax, liquid isoparaffin, liquid paraffin, mineral oil and vaseline;

natural fats and oils, such as natural waxes including jojoba oil, carnauba wax, candelilla wax, rice wax, shellac, lanolin, mink oil wax, spermaceti, sugarcane wax, sperm oil, beeswax and montan wax; avocado oil, almond oil, olive oil, extra virgin olive oil, sesame oil, rice bran oil, rice oil, rice germ oil, corn oil, soybean oil, maize oil, persic oil, palm kernel oil, palm oil, castor oil, grape seed oil, cottonseed oil, coconut oil, hydrogenated coconut oil, tallow, hydrogenated oil, horse oil, mink oil, egg yolk oil, egg yolk fatty oil, rose hip oil, candlenut oil, evening primrose oil, wheat germ oil, peanut oil, tsubaki oil, sasanqua oil, cacao butter, Japan wax, beef bone fat, neatsfoot oil, lard, horse fat, mutton tallow, shea butter, macadamia nut oil and meadowfoam seed oil;

fatty acids such as lauric acid, myristic acid, palmitic acid, stearic acid, behenic acid, oleic acid, isostearic acid, 12-hydroxystearic acid, undecylenic acid and coconut fatty acid;

higher alcohols such as isostearyl alcohol, octyldodecanol, hexyldecanol, cholesterol, phytosterol, lauryl alcohol, myristyl alcohol, cetanol, stearyl alcohol, oleyl alcohol, behenyl alcohol and cetostearyl alcohol;

alkyl glyceryl ethers such as batyl alcohol, chimyl alcohol, selachyl alcohol and isostearyl glyceryl ether;

esters such as isopropyl myristate, butyl myristate, isopropyl palmitate, ethyl stearate, butyl stearate, ethyl oleate, ethyl linoleate, isopropyl linoleate, cetyl caprylate, hexyl laurate, isooctyl myristate, decyl myristate, myristyl myristate, cetyl myristate, octadecyl myristate, cetyl palmitate, stearyl stearate, decyl oleate, oleyl oleate, cetyl ricinoleate, isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, octyldodecyl ricinoleate,
ethyl isostearate, isopropyl isostearate, cetyl 2-ethylhexanoate, cetostearyl 2-ethylhexanoate, stearyl 2-ethylhexanoate, hexyl isostearate, ethylene glycol dioctanoate, ethylene glycol dioleate, propylene glycol dicaprylate, propylene glycol di(caprylate caprate), propylene glycol dicaprate, propylene glycol dioleate, neopentyl glycol dicaprate, neopentyl glycol dioctanoate, glyceryl tricaprylate, glyceryl tri-2-ethylhexanoate, glyceryl tri(caprylate caprate), glyceryl tri(caprylate caprate stearate), glyceryl triundecylate, glyceryl triisopalmitate, glyceryl triisostearate, trimethylolpropane tri-2-ethylhexanoate, trimethylolpropane triisostearate, pentaerythrityl tetra-2-ethylhexanoate, pentaerythrityl tetramyristate,
pentaerythrityl tetraisostearate, diglyceryl tetraisostearate, octyldodecyl neopentanoate, isocetyl octanoate, isostearyl octanoate, 2-ethylhexyl isopelargonate, hexyldecyl dimethyloctanoate, octyldodecyl dimethyloctanoate, 2-ethylhexyl isopalmitate, isocetyl isostearate, isostearyl isostearate, octyldodecyl isostearate, lauryl lactate, myristyl lactate, cetyl lactate, octyldodecyl lactate, triethyl citrate, acetyltriethyl citrate, acetyltributyl citrate, trioctyl citrate, triisocetyl citrate, trioctyldodecyl citrate, diisostearyl malate, 2-ethylhexyl hydroxystearate, di-2-ethylhexyl succinate, diisopropyl adipate, diisobutyl adipate, dioctyl adipate, diheptylundecyl adipate, diethyl sebacate, diisopropyl sebacate, dioctyl sebacate,
cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoylhydroxystearate, stearyl 12-stearoylhydroxystearate, isostearyl 12-stearoylhydroxystearate, polyoxyethylene (3) polyoxypropylene (1) cetyl ether acetate, polyoxyethylene (3) polyoxypropylene (1) isocetyl ether acetate, isononyl isononanoate, octyl isononanoate, tridecyl isononanoate and isotridecyl isononanoate;

silicone oils such as methyl polysiloxane, methyl phenyl polysiloxane, methyl hydrogen polysiloxane, methyl cyclopolysiloxane, octamethyl cyclotetrasiloxane, decamethyl cyclopentasiloxane, dodecamethyl cyclohexasiloxane, octamethyl trisiloxane, decamethyl tetrasiloxane, tetradecamethyl hexasiloxane, highly polymerized methyl polysiloxane, dimethyl siloxane/methyl (polyoxyethylene) siloxane/methyl (polyoxypropylene) siloxane copolymer, dimethyl siloxane/methyl (polyoxyethylene) siloxane copolymer, dimethyl siloxane/methyl (polyoxypropylene) siloxane copolymer, dimethyl siloxane/methyl cetyloxysiloxane copolymer, dimethyl siloxane/methyl stearoxysiloxane copolymer, polyether-modified silicones, alcohol-modified silicones, alkyl-modified silicones and amino-modified silicones;

polymers such as sodium alginate, carrageenan, agar, furcelleran, guar gum, quince seed, konjac mannan, tamarind gum, tara gum, dextrin, starch, locust bean gum, gum arabic, ghatti gum, karaya gum, tragacanth gum, arabinogalactan, pectin, marmelo, chitosan, starch, curdlan, xanthan gum, gellan gum, cyclodextrin, dextran, pullulan, microcrystalline cellulose, methylcellulose, ethylcellulose, hydroxyethylcellulose, hydroxypropylcellulose, hydroxypropylmethylcellulose, carboxymethylcellulose, carboxy starch, cationized cellulose, starch phosphate, cationized guar gum, carboxymethyl/hydroxypropylated guar gum, hydroxypropylated guar gum, albumin, casein, gelatin, sodium polyacrylate, poly(acrylamide), carboxyvinyl polymers, polyethyleneimine,
highly polymerized polyethylene glycol, polyvinyl alcohol, polyvinylpyrrolidone, polyvinyl ether, polyacrylamide, acrylic acid copolymers, methacrylic acid copolymers, maleic acid copolymers, vinylpyridine copolymers, ethylene/acrylic acid copolymers, vinylpyrrolidone polymers, vinyl alcohol/vinylpyrrolidone copolymers, nitrogen-substituted acrylamide polymers, amino-modified silicones, cationized polymers, dimethylacryl ammonium polymers, acrylate anionic polymers, methacrylate anionic polymers, modified silicones, acrylic acid/alkyl (C₁₀-C₃₀) methacrylate copolymers and polyoxyethylene/polyoxypropylene copolymer;

lower alcohols such as ethanol, isopropyl alcohol, 1-butanol, 2-butanol and benzyl alcohol;

polyhydric alcohols such as ethylene glycol, diethylene glycol, polyethylene glycol, propylene glycol, polypropylene glycol, glycerin, diglycerin, polyglycerin, 1,3-butanediol, triethylene glycol, dipropylene glycol, 3-methyl-1,3-butanediol, 1,2-pentanediol, 1,4-pentanediol, 1,5-pentanediol, 2,4-pentanediol, 2-methyl-2,4-pentanediol, 3-methyl-1, 5-pentanediol, 1,2-hexanediol and 1, 6-hexanediol;

anionic surfactants such as potassium cocoate, sodium cocoate, triethanolamine cocoate, potassium laurate, sodium laurate, triethanolamine laurate, potassium myristate, sodium myristate, isopropanolamine myristate, potassium palmitate, sodium palmitate, isopropanolamine palmitate, potassium stearate, sodium stearate, triethanolamine stearate, potassium oleate, sodium oleate, sodium castor oil fatty acid, zinc undecylenate, zinc laurate, zinc myristate, magnesium myristate, zinc palmitate, zinc stearate, calcium stearate, magnesium stearate, aluminum stearate, calcium myristate, magnesium myristate, aluminum dimyristate, aluminum isostearate,
polyoxyethylene lauryl ether acetic acid, sodium polyoxyethylene lauryl ether acetate, polyoxyethylene tridecyl ether carboxylic acid, sodium polyoxyethylene tridecyl ether acetate, sodium stearoyl lactate, sodium isostearoyl lactate, sodium N-lauroyl sarcosinate, cocoyl sarcosine, sodium N-cocoyl sarcosinate, triethanolamine N-cocoyl sarcosinate, lauroyl sarcosine, potassium lauroyl sarcosinate, triethanolamine lauroyl sarcosinate, oleoyl sarcosine, sodium myristoyl sarcosinate, sodium stearoyl glutamate, cocoyl glutamic acid, potassium cocoyl glutamate, sodium cocoyl glutamate, triethanolamine cocoyl glutamate, lauroyl acylglutamic acid, potassium lauroyl acylglutamate,
sodium lauroyl acylglutamate, triethanolamine lauroyl acylglutamate, myristoyl acylglutamic acid, potassium myristoyl acylglutamate, sodium myristoyl acylglutamate, stearoyl acylglutamic acid, potassium stearoyl acylglutamate, disodium stearoyl acylglutamate, sodium hydrogenated tallow glutamate, sodium cocoyl glutamate/hydrogenated tallow glutamate, sodium N-methyl N-(1-oxococonut alkyl) β-alanine, lauroyl methyl alanine, sodium lauroyl methyl alanine, triethanolamine lauroyl methyl alanine, sodium myristoyl methyl alanine, sodium lauroyl methyl taurate, potassium N-cocoyl-N-methyl taurate, sodium N-cocoyl-N-methyl taurate, magnesium cocoyl methyl taurate, sodium myristoyl methyl taurate,
sodium palmitoyl methyl taurate, sodium stearoyl methyl taurate, sodium oleoyl methyl taurate, sodium alkane sulfonate, sodium tetradecenesulfonate, sodium dioctyl sulfosuccinate, disodium monolauryl sulfosuccinate, sodium cocoyl ethyl ester sulfonate, sodium lauryl sulfate, triethanolamine lauryl sulfate, sodium cetyl sulfate, triethanolamine alkyl (11, 13, 15) sulfate, sodium alkyl (12, 13) sulfate, triethanolamine alkyl (12, 13) sulfate, ammonium alkyl (12, 14, 16) sulfate, diethanolamine alkyl (12, 13) sulfate, triethanolamine alkyl (12-14) sulfate, triethanolamine alkyl (12-15) sulfate, magnesium ·triethanolamine coco sulfate, ammonium lauryl sulfate, potassium lauryl sulfate, magnesium lauryl sulfate, monoethanolamine lauryl sulfate, diethanolamine lauryl sulfate,
sodium myristyl sulfate, sodium stearyl sulfate, sodium oleyl sulfate, triethanolamine oleyl sulfate, sodium polyoxyethylene lauryl ether sulfate, triethanolamine polyoxyethylene lauryl ether sulfate, sodium polyoxyethylene (1) alkyl (11, 13, 15) ether sulfate, triethanolamine polyoxyethylene (1) alkyl (11, 13, 15) ether sulfate, sodium polyoxyethylene (3) alkyl (11-15) ether sulfate, sodium polyoxyethylene (2) alkyl (12, 13) ether sulfate, sodium polyoxyethylene (3) alkyl (12-14) ether sulfate, sodium polyoxyethylene (3) alkyl (12-15) ether sulfate, sodium polyoxyethylene (2) lauryl ether sulfate, sodium polyoxyethylene (3) myristyl ether sulfate, sodium higher fatty acid alkanolamide sulfate, lauryl monophosphate, sodium lauryl phosphate,
potassium cetyl phosphate, diethanolamine cetyl phosphate, polyoxyethylene oleyl ether phosphate, polyoxyethylene lauryl ether phosphate, sodium polyoxyethylene lauryl ether phosphate, polyoxyethylene cetyl ether phosphate, sodium polyoxyethylene cetyl ether phosphate, polyoxyethylene stearyl ether phosphate, polyoxyethylene oleyl ether phosphate, sodium polyoxyethylene oleyl ether phosphate, polyoxyethylene alkylphenyl ether phosphate, sodium polyoxyethylene alkylphenyl ether phosphate, triethanolamine polyoxyethylene alkylphenyl ether phosphate, polyoxyethylene octyl ether phosphate, polyoxyethylene (10) alkyl (12, 13) ether phosphate, polyoxyethylene alkyl (12-15) ether phosphate, polyoxyethylene alkyl (12-16) ether phosphate, triethanolamine polyoxyethylene lauryl ether phosphate and diethanolamine polyoxyethylene oleyl ether phosphate;

cationic surfactants such as dioctylamine, dimethylstearylamine, trilaurylamine, N-[2-(diethylamino)ethyl] octadecanamide, lauryl trimethyl ammonium chloride, cetyl trimethyl ammonium chloride, cetyl trimethyl ammonium bromide, cetyl trimethyl ammonium saccharinate, stearyl trimethyl ammonium chloride, alkyl (20-22) trimethyl ammonium chloride, lauryl trimethyl ammonium bromide, alkyl (16, 18) trimethyl ammonium chloride, stearyl trimethyl ammonium bromide, stearyl trimethyl ammonium saccharinate, alkyl (28) trimethyl ammonium chloride, di(polyoxyethylene) oleyl methyl ammonium chloride (2EO), dipolyoxyethylene stearyl methyl ammonium chloride, polyoxyethylene (1) polyoxypropylene (25) diethyl methyl ammonium chloride, tri(polyoxyethylene) stearyl ammonium chloride (5EO), distearyl dimethyl ammonium chloride,
dialkyl (12-15) dimethyl ammonium chloride, dialkyl (12-18) dimethyl ammonium chloride, dialkyl (14-18) dimethyl ammonium chloride, dicocoyl dimethyl ammonium chloride, dicetyl dimethyl ammonium chloride, isostearyl lauryl dimethyl ammonium chloride, benzalkonium chloride, myristyl dimethyl benzyl ammonium chloride, lauryl dimethyl (ethylbenzyl) ammonium chloride, stearyl dimethyl benzyl ammonium chloride, lauryl pyridinium chloride, cetyl pyridinium chloride, lauroyl colamino formyl methyl pyridinium chloride, stearoyl colamino formyl methyl pyridinium chloride, alkyl isoquinolium bromide, methyl benzethonium chloride and benzethonium chloride;

amphoteric surfactants such as 2-alkyl-N-carboxymethyl-N-hydroxyethyl imidazolinium betaine, alkyldiaminoethylglycine hydrochloride, sodium lauryl diaminoethyl glycinate, sodium undecyl hydroxyethyl imidazoliium betaine, undecyl-N-carboxymethyl imidazoliium betaine, disodium cocoyl-N-carboxyethyl-N-hydroxyethyl ethylenediamine, disodium cocoyl-N-carboxyethoxyethyl-N-carboxyethyl ethylenediamine, disodium cocoyl-N-carboxymethoxyethyl-N-carboxymethyl ethylenediamine, sodium laurylaminopropionate, sodium laurylaminodipropionate, triethanolamine laurylaminopropionate, sodium palm kernel oil fatty acid acyl-N-carboxyethyl-N-hydroxyethylethylenediamine, lauryl dimethylaminoacetic acid betaine, cocoalkyl dimethylaminoacetic acid betaine, stearyl dimethyl glycine, sodium stearyl dimethyl glycine, cocoyl amide propyldimethyl glycine, palm kernel oil amide propyl dimethyl glycine, lauric acid amide betaine propylacetate, propyl betaine ricinoleate amide, stearyl dihydroxyethyl glycine and lauryl hydroxy sulfobetaine;

natural surfactants such as saponin, lecithin, soybean phospholipid, hydrogenated soybean phospholipid, soybean lysophospholipid, hydrogenated soybean lysophospholipid, egg yolk lecithin, hydrogenated egg yolk lysophosphatidylcholine, phosphatidylcholine, phosphatidylethanolamine, phosphatidylserine, sphingophospholipid, sphingomyelin, ganglioside, bile acid, cholic acid, deoxycholic acid, sodium cholate, sodium deoxycholate, spiculisporic acid, rhamnolipid, trehalose lipid, sophorolipid and mannosylerythritol lipid;

ultraviolet absorbers, including para-aminobenzoic acid derivatives such as para-aminobenzoic acid, ethyl para-aminobenzoate, glyceryl para-aminobenzoate, amyl para-dimethylaminobenzoate and 2-ethylhexyl para-dimethylaminobenzoate, cinnamic acid derivatives such as benzyl cinnamate, glyceryl 2-ethylhexanoate di-para-methoxy cinnamate, 2,4-diisopropyl methyl cinnamate, 2,4-diisopropyl ethyl cinnamate, potassium para-methoxycinnamate, sodium para-methoxycinnamate, isopropyl para-methoxycinnamate, 2-ethylhexyl para-methoxycinnamate, 2-ethoxyethyl paramethoxycinnamate and ethyl para-ethoxycinnamate, urocanic acid derivatives such as urocanic acid and ethyl urocanate,
benzophenone derivatives such as 2,4-dihydroxybenzophenone, 2,2',4,4'-tetrahydroxybenzophenone, sodium 2-hydroxy-4-methoxy-5-sulfobenzophenone, 2-hydroxy-4-methoxybenzophenone-5-sulfonic acid, 2-hydroxy-4-methoxybenzophenone, 2,2'-dihydroxy-4,4'-dimethoxybenzophenone and sodium 2,2'-dihydroxy-4,4'-dimethoxy-5-sulfobenzophenone, salicylic acid derivatives such as ethylene glycol salicylate, 2-ethylhexyl salicylate, phenyl salicylate, benzyl salicylate, p-tert-butylphenyl salicylate, homomenthyl salicylate and 3,3,5-trimethylcyclohexyl salicylate, 2-(2'-hydroxy-5'-methoxyphenyl)benzotriazole and 4-tert-butyl-4'-methoxybenzoylmethane;

powders and color materials such as kaolin, silicic anhydride, aluminum magnesium silicate, sericite, talc, boron nitride, mica, montmorillonite, ramie cellulose powder, wheat starch, silk powder, cornstarch, nitro dye, azo dye, nitroso dye, triphenylmethane dye, xanthene dye, quinoline dye, anthraquinone dye, indigo dye, pyrene dye, phthalocyanine dye,
natural dyes including flavonoid, quinone, porphyrin, water-soluble annatto, cuttlefish ink powder, caramel, guaiazulene, gardenia blue, gardenia yellow, cochineal, shikonin, sodium copper chlorophyllin, paprika dye, safflower red, safflower yellow, laccaic acid and riboflavin butyrate, carbon black, yellow iron oxide, black iron oxide, red iron oxide, iron blue, ultramarine blue, zinc oxide, chromium oxide, titanium oxide, black titanium oxide, zirconium oxide, chromium hydroxide, alumina, magnesium oxide, barium sulfate, aluminum hydroxide, calcium carbonate, lithium cobalt titanate, manganese violet and pearl pigment;

plant extracts such as angelica extract, gambir extract, avocado extract, hydrangea extract, gynostemma pentaphyllum extract, althea extract, arnica extract, oil-soluble arnica extract, almond extract, aloe extract, styrax resin extract, ginkgo extract, nettle extract, orris extract, fennel extract, turmeric extract, rose fruit extract, echinacea leaf extract, scutellaria root extract, phellodendron bark extract, Japanese coptis extract, barley extract, okura extract, hypericum extract, oil-soluble hypericum extract, white nettle extract, oil-soluble white nettle extract, restharrow extract, watercress extract, orange flower water, persimmon tannin, pueraria root extract, Japanese valerian extract, cattail extract, chamomile extract, oil-soluble chamomile extract, chamomile distillate, oat extract, carrot extract, oil-soluble carrot extract, carrot oil, artemisia capillaris extract, glycyrrhiza extract, glycyrrhiza extracted powder, glycyrrhiza flavonoid, cantharides tincture, raspberry extract, kiwi extract, cinchona extract,
cucumber extract, apricot kernel extract, quince seed extract, gardenia extract, sasa albo-marginata extract, sophora root extract, walnut shell extract, clematis extract, black sugar extract, chlorella extract, mulberry root extract, cinnamon bark extract, gentian extract, geranium herb extract, black tea extract, nuphar extract, burdock root extract, oil-soluble burdock root extract, wheat germ extract, hydrolyzed wheat powder, rice bran extract, fermented rice bran extract, comfrey extract, asiasarum root extract, saffron extract, saponaria extract, oil-soluble salvia extract, crataegus fruit extract, zanthoxylum fruit extract, shiitake mushroom extract, shiitake mushroom extracted powder, rehmannia root extract, lithospermum root extract, oil-soluble lithospermum root extract, perilla herb extract, linden extract, oil-soluble linden extract, filipendula extract, peony root extract, Job's tears extract, ginger extract, oil-soluble ginger extract, ginger tincture, calamus rhizome extract, birch extract, oil-soluble birch extract, birch sap, honeysuckle extract, horsetail extract, oil-soluble horsetail extract,
scordinine, stevia extract, ivy extract, crataegus extract, sambucus extract, juniper extract, yarrow extract, oil-soluble yarrow extract, peppermint extract, sage extract, oil-soluble sage extract, sage water, mallow extract, celery extract, cnidium rhizome extract, cnidium rhizome water, swertia herb extract, soy extract, jujube extract, thyme extract, green tea extract, tea leaf dry distilled solution, tea seed extract, clove extract, citrus unshiu peel extract, camellia extract, centella extract, oil-soluble walnut extract, duke extract, terminalia extract, Japanese angelica root extract, oil-soluble Japanese angelica root extract, Japanese angelica root water, calendula extract, oil-soluble calendula extract, soy milk powder, peach seed extract, bitter orange peel extract, houttuynia extract, tomato extract, tormentilla extract, natto extract, ginseng extract, oil-soluble ginseng extract, garlic extract, wild rose extract,
oil-soluble wild rose extract, malt extract, malt root extract, ophiopogon tuber extract, parsley extract, barley leaf juice concentrate, peppermint distillate, witch hazel distillate, witch hazel extract, rose extract, pellitory extract, isodonis extract, loquat leaf extract, oil-soluble loquat leaf extract, coltsfoot extract, hoelen extract, butcher broom extract, butcher broom extracted powder, grape extract, grape leaf extract, grape water, hayflower extract, sponge gourd extract, sponge gourd solution, safflower extract, oil-soluble linden extract, linden water, paeonia extract, hop extract, oil-soluble hop extract, pine extract, milk thistle extract, horse chestnut extract, oil-soluble horse chestnut extract, mukurossi peel extract, balm mint extract, sweet clover extract, peach leaf extract, oil-soluble peach leaf extract, bean sprouts extract, cornflower extract, cornflower distillate, eucalyptus extract, saxifrage extract, lily extract, coix extract, oil-soluble coix extract, mugwort extract, Japanese mugwort water, lavender extract, lavender water, apple extract, ganoderma extract, lettuce extract, Chinese milk vetch extract, rose water, rosemary extract, oil-soluble rosemary extract, Romanchamomile extract and burnet extract;

amino acids and peptides such as glycine, valine, leucine, isoleucine, serine, threonine, phenylalanine, tyrosine, tryptophan, cystine, cysteine, methionine, hydroxyproline, aspartic acid, asparagine, glutamic acid, glutamine, histidine, γ-aminobutyric acid, DL-pyrrolidonecarboxylic acid, ε-aminocaproic acid, hydrolyzed elastin, water-soluble elastin, hydrolyzed collagen, water-soluble collagen, casein, glutathione, wheat peptide and soybean peptide;

vitamins and vitamin-like substances, including vitamin A such as retinol, retinal, retinoic acid, retinol acetate and retinol palmitate, carotenoids such as α-carotene, β-carotene, γ-carotene, δ-carotene, lycopene, zeaxanthin, cryptoxanthin, echinenone and astaxanthin, vitamin B1 such as thiamines, vitamin B2 such as riboflavin, vitamin B6 such as pyridoxine, pyridoxal and pyridoxamine, vitamin B12 such as cyanocobalamin,
vitamin C such as folic acids, nicotinic acid, nicotinamide, pantothenic acids, biotins, L-ascorbic acid, sodium L-ascorbate, L-ascorbyl stearate, L-ascorbyl palmitate, L-ascorbyl dipalmitate, L-ascorbyl tetraisopalmitate, disodium L-ascorbic acid sulfate, L-ascorbyl magnesium, sodium L-ascorbyl phosphate, ascorbic acid-2-phosphate and L-ascorbic acid-2-glucoside, vitamin D such as ergocalciferol and cholecalciferol, vitamin E such as d-α-tocopherol, DL-α-tocopherol, dl-α-tocopherol acetate, dl-α-tocopherol succinate, β-tocopherol, γ-tocopherol and d-δ-tocopherol, ubiquinones, vitamin K, carnitine, ferulic acid, γ-oryzanol, α-lipoic acid and orotic acid;

preservative agents such as benzoic acid, sodium benzoate, undecylenic acid, salicylic acid, sorbic acid, potassium sorbate, dehydroacetic acid, sodium dehydroacetate, isobutyl paraoxybenzoate, isopropyl paraoxybenzoate, ethyl paraoxybenzoate, butyl paraoxybenzoate, propyl paraoxybenzoate, benzyl paraoxybenzoate, methyl paraoxybenzoate, sodium methyl paraoxybenzoate, phenoxyethanol, photosensitizing dye No. 101, photosensitizing dye No. 201 and photosensitizing dye No. 401;

antioxidants such as butylhydroxyanisol, butylhydroxytoluene, propyl gallate, erythorbic acid, sodium erythorbate, para-hydroxyanisol and octyl gallate;

sequestering agents, including metal ionic compounds such as trisodium hydroxyethyl ethylenediamine triacetate, edetic acid, disodium edetate, trisodium edetate, tetrasodium edetate, sodium citrate, gluconic acid, phytic acid, sodium polyphosphate and sodium metaphosphate;

moisturizers such as hyaluronic acid, sodium hyaluronate, sodium chondroitin sulfate, sodium lactate, sodium pyrrolidone carboxylate, betaine, lactic acid bacteria culture, yeast extract and ceramide;

anti-inflammatory agents such as glycyrrhizinic acid, trisodium glycyrrhizinate, dipotassium glycyrrhizinate, monoammonium glycyrrhizinate, β-glycyrrhetinic acid, glyceryl glycyrrhetinate, stearyl glycyrrhetinate, lysozyme chloride, hydrocortisone and allantoin;

pH adjusters such as sodium hydroxide, potassium hydroxide and triethanolamine;
salts such as sodium chloride, potassium chloride, magnesium chloride and sodium sulfate;

α-hydroxy acids such as citric acid, glycolic acid, tartaric acid and lactic acid;

whitening agents such as arbutin, α-arbutin and placental extract;

essential oils such as angelica oil, ylang ylang oil, elemi oil, chamomile oil, Romanchamomile oil, cardamom oil, calamus oil, galbanum oil, camphor oil, carrot seed oil, clary sage oil, clove oil, cinnamon oil, coriander oil, cypress oil, sandalwood oil, cedarwood oil, citronella oil, cinnamon leaf oil, jasmine absolute, juniperberry oil, ginger extract, spearmint oil, sage oil, cedar oil, geranium oil, thyme oil, tea tree oil, nutmeg oil, niaouli oil, neroli oil, pine oil, basil oil, mentha oil, patchouli oil, palmarosa oil, fennel oil, petitgrain oil, black pepper oil, frankincense oil, vetivert oil, peppermint oil, bergamot oil, benzoin oil, aniba rosaeodora oil, marjoram oil, myrrh oil, balm mint oil, eucalyptus oil, ravensara oil, lavandin oil, lavender oil, linden oil, rose oil, rosewood oil, rosemary oil and lovage oil;

terpenes such as pinene, terpinene, terpinolene, myrcene and longifolene;
perfumes and water.

The formulation types and forms of the emulsified skin external preparations and the cosmetics of the present invention are not particularly limited as long as they are used directly on skin. In a preferred embodiment, the emulsified skin external preparations and cosmetics are applied to the skin in the vicinity of where subcutaneous fat metabolism is desired.

In the broad sense, the emulsified skin external preparations and the cosmetics include any formulations that are used directly on skin, such as skin milks, skin creams, foundation creams, massage creams, cleansing creams, shaving creams, cleansing foams, skin toners, lotions, packs, lipsticks, rouges, eye shadows, manicures, soaps, body shampoos, hand soaps, shampoos, rinses, hair tonics, treatment conditioners, hair creams, hair sprays, hair growth tonics, baldness remedies, hairdyes, styling spritz, depilatories, antidandruff agents, toothpastes, denture adhesives, mouthwashes, permanent waving agents, curling agents, styling agents, ointments, adhesive skin patches, taping agents, bath agents, antiperspirants and sunscreen agents. The emulsified skin external preparations and the cosmetics may be used regardless of user's gender and age, and may be used for animal skin as well as human skin.

The emulsified skin external preparations and the cosmetics according to the present invention may be in any forms including solids, liquids, semisolids, powders, granules, tablets, gels, foams and sprays.

Although not particularly limited, the formulation types and forms containing aqueous media are preferably selected because the present invention is particularly effective in improving the poor emulsification stability of the conventional carnitine derivatives and salts thereof in aqueous formulations. In this case, water may be usually used in an amount of 0.01 to 99.99% by mass of the emulsified skin external preparations or cosmetics of the present invention.

The cosmetics according to the present invention may further contain existing cosmetic ingredients. For example, all of the cosmetic ingredients listed in The Japanese Standards of Cosmetic Ingredients 2nd edition (edited by Pharmaceutical and Medical Device Regulatory Science Society of Japan and published by Yakuji Nippo, Ltd. (1984)), The Japanese Cosmetic Ingredients Codex (edited by Ministry of Health and Welfare, Pharmaceutical Affairs Bureau, Examination Division and published by Yakuji Nippo, Ltd. (1993)), Supplement to The Japanese Cosmetic Ingredients Codex (edited by Ministry of Health and Welfare, Pharmaceutical Affairs Bureau, Examination Division and published by Yakuji Nippo, Ltd. (1993)), The Comprehensive Licensing Standards of Cosmetics by Category (edited by Ministry of Health and Welfare, Pharmaceutical Affairs Bureau, Examination Division and published by Yakuji Nippo, Ltd. (1993)), The Japanese Cosmetic Ingredients Codex by Category (edited by Ministry of Health and Welfare, Pharmaceutical Affairs Bureau, Examination Division and published by Yakuji Nippo, Ltd. (1997)), Keshouhin Genryou Jiten (Dictionary of Cosmetic Ingredients) (Nikko Chemicals., Co. Ltd. (1991)), and Atarashii Keshouhin Kinou Sozai 300 (300 Latest Cosmetic Functional Materials) (CMC Publishing Co., Ltd. (2002)) can be used while still achieving the advantageous effects of the present invention.

The emulsified skin external preparations and the cosmetics of the present invention may be produced by common methods depending on the formulation types, for example by dissolving, mixing or dispersing the aforesaid ingredients in predetermined amounts.

### EXAMPLES

The present invention will be described in greater detail based on examples below. However, it should be construed that the invention is not limited to such examples. In Examples, % is percent by mass unless otherwise specified.

### [Synthetic Example 1]

### Synthesis of L-carnitine 2-hexyldecanoate hydrochloride

In an ice bath, 45.6 g (0.283 mol) of L-carnitine was dissolved in 150 ml of trifluoroacetic acid. To the resultant solution, 116.6 g (0.425 mol) of 2-hexyldecanoic acid chloride was added dropwise over a period of 10 minutes, followed by stirring at 80°C for 4 hours. The solvent was distilled away under reduced pressure. The resultant dark brown oily substance weighing 264.6 g was washed with 200 ml of n-hexane three times, and 200 ml of a black oily substance was obtained. A 3.0 g portion of the oily substance was subj ected to extraction with 20 ml of ethanol, 30 ml of n-butanol and 60 ml of water. The organic phase obtained was washed with 60 ml of water, and with a mixture of 20 ml of ethanol and 60 ml of water. The obtained organic phase was further washed with 60 ml of water, collected and dried. Evaporating the solvent resulted in a yield of 2.0 g of L-carnitine 2-hexyldecanoate hydrochloride.

The structure of the target compound was identified by NMR, liquid chromatography-mass spectrometry (LC/MS) and elemental analysis as follows.

### (NMR)

¹H-NMR (CDCl₃): 0.90 ppm (6H, t, 7.0 Hz), 1.29-1.62 ppm (24H, m), 2.41 ppm (1H, m), 2.76 ppm (2H, d, 6.0 Hz), 3.21 ppm (9H, s), 3.75 ppm (1H, d, 14.6 Hz), 3.92 ppm (1H, dd, 8.2, 14.6 Hz), 5.60-5.64 ppm (1H, m)
NMR apparatus: Burker Advance 500
Sample concentration: 40 mg sample/422 mg deuterated chloroform
Temperature: room temperature.

### (Liquid chromatography-mass spectrometry (LC/MS))

MS (ESI) m/z: 400.5 [M⁺] (LC/MS)
Liquid chromatograph (LC): Agilent 1100 series Column: Shodex silica C8-5B
Column temperature: 40°C
Eluting solution: 20 mM aqueous ammonium acetate solution/acetonitrile = 30/70
Flow rate: 1.0 ml/min
Sample concentration and injection amount: 10 µl x 5 mg/ml eluting solution
Detection: photodiode array UV 200-700 nm
Mass spectrometer (MS): Thermoquest LCQ Advantage
Ionizing method: ESI (electrospray ionization)
Scan range: m/z 50-1000 (alternate positive and negative charges)
MS/MS collision energy: 40%.

### (Elemental analysis)

Elemental analysis: C: 62.8%, H: 10.6%, N: 3.5%, O: 14.9%, Cl: 8.2%
C, H, N and O: Organic elemental analyzer CHNS-932 and oxygen analysis option VTF-900 (manufactured by LECO)
References: sym-diphenylthiourea (for analysis of C, H and N), and p-nitroaniline (for analysis of O)
Cl: ion chromatography (Exactly 1 mg of the sample was weighed and mixed with an eluting solution (1.8 mM Na₂CO₃ + 1.7 mM NaHCO₃) to a constant volume of 100 ml, and analyzed with an anion chromatograph (DIONEX DX-500) to determine Cl in the sample.)
Column: Shodex SI-90 4E
Flow rate: 1.0 ml/min
Injection amount: 25 µl
Detector: Electrical conductivity detector Suppressor: ASRS-I.

### [Synthetic Example 2]

### Synthesis of L-carnitine 2-methylpalmitate hydrochloride

In an ice bath, 45.6 g (0.283 mol) of L-carnitine was dissolved in 150 ml of trifluoroacetic acid. To the resultant solution, 122.4 g (0.425 mol) of 2-methylpalmitic acid chloride was added dropwise over a period of 10 minutes, followed by stirring at 80°C for 4 hours. The solvent was distilled away under reduced pressure. The resultant dark brown oily substance weighing 278.0 g was washed with 200 ml of n-hexane three times, and 200 ml of a black oily substance was obtained. A 3.0 g portion of the oily substance was subj ected to extraction with 20 ml of ethanol, 30 ml of n-butanol and 60 ml of water. The organic phase obtained was washed with 60 ml of water, and with a mixture of 20 ml of ethanol and 60 ml of water. The obtained organic phase was further washed with 60 ml of water, collected and dried. Evaporating the solvent resulted in a yield of 2.2 g of L-carnitine 2-methylpalmitate hydrochloride.

The structure of the target compound was identified by NMR, liquid chromatography-mass spectrometry (LC/MS) and elemental analysis as described in Synthetic Example 1, the results being shown below.

### (NMR)

¹H-NMR (CDCl₃): 2.50 ppm (1H, m), 2.75 ppm (1H, dd, 6.2, 1.5 Hz), 2.80 ppm (1H, m), 3.73 ppm (1H, d, 14.4 Hz), 3. 91 ppm (1H, dd, 14.4, 8.5 Hz), 5.61-5.65 ppm (1H, m).

### <Liquid chromatography-mass spectrometry (LC/MS)>

MS (ESI) m/z: 414.5 [M⁺]

### (Elemental analysis)

C: 64.3%, H: 10.3%, N: 3.2%, O: 14.2%, Cl: 8.0%.

### [Synthetic Example 3]

### Synthesis of L-carnitine 2-butyloctanoate hydrochloride

In an ice bath, 45.6 g (0.283 mol) of L-carnitine was dissolved in 150 ml of trifluoroacetic acid. To the resultant solution, 92.7 g (0.425 mol) of 2-butyloctanoic acid chloride was added dropwise over a period of 10 minutes, followed by stirring at 80°C for 4 hours. The solvent was distilled away under reduced pressure. The resultant dark brown oily substance weighing 250.0 g was washed with 200 ml of n-hexane three times, and 200 ml of a black oily substance was obtained. A 3.0 g portion of the oily substance was subjected to extraction with 20 ml of ethanol, 30 ml of n-butanol and 60 ml of water. The organic phase obtained was washed with 60 ml of water, and with a mixture of 20 ml of ethanol and 60 ml of water. The obtained organic phase was further washed with 60 ml of water, collected and dried. Evaporating the solvent resulted in a yield of 1.6 g of L-carnitine 2-butyloctanoate hydrochloride.

The structure of the target compound was identified by NMR, liquid chromatography-mass spectrometry (LC/MS) and elemental analysis as described in Synthetic Example 1, the results being shown below.

### (NMR)

¹H-NMR (CDCl₃): 2.76 ppm (1H, d, 6.0 Hz), 2.80 ppm (1H, m), 3.76 ppm (1H, d, 14.4 Hz), 3.94 ppm (1H, dd, 14.4, 8.2 Hz), 5.60-5.64 ppm (1H, m).

### (Liquid chromatography-mass spectrometry (LC/MS))

MS (ESI) m/z: 344.5 [M⁺]

### (Elemental analysis)

C: 60.1%, H: 9.5%, N: 3.5%, O: 16.7%, Cl: 10.2%.

### [Formulation Examples 1 to 7]

Cosmetics were prepared according to the compositions shown in Table 1 below. Glyceryl fatty acid esters and polyoxyethylene sorbitan fatty acid esters were used as nonionic surfactants.

Ingredients were blended according to the compositions described in the columns for cosmetics 1 to 7 in Table 1 below, and were homogeneously suspended and dissolved while heating to 85°C to give liquids A, B and C. While the liquid A was stirred with a homomixer, the liquid B and the liquid C were gradually added thereto in this order. The mixtures were continuously stirred while being ice-cooled to approximately 30°C, and were thereafter naturally cooled to room temperature to give cosmetics 1 to 7.

### [Table 1]

**Table 1**

| | Ingredients | Amounts (%) | | | | | | |
|---|---|---|---|---|---|---|---|---|
| | | Cosmetic 1 | Cosmetic 2 | Cosmetic 3 | Cosmetic 4 | Cosmetic 5 | Cosmetic 6 | Cosmetic 7 |
| Liquid A | POE (20) sorbitan monostearate | 1.0 | 1.0 | 1.0 | 1.0 | 1.0 | 1.0 | 1.0 |
| | POE (40) sorbitan tetraoleate | 1.5 | 1.5 | 1.5 | 1.5 | 1.5 | 1.5 | 1.5 |
| | Glyceryl stearate | 1.0 | 1.0 | 1.0 | 1.0 | 1.0 | 1.0 | 1.0 |
| | Stearic acid | 0.5 | 0.5 | 0.5 | 0.5 | 0.5 | 0.5 | 0.5 |
| | Behenyl alcohol | 3.0 | 3.0 | 3.0 | 3.0 | 3.0 | 3.0 | 3.0 |
| | Cetyl palmitate | 0.5 | 0.5 | 0.5 | 0.5 | 0.5 | 0.5 | 0.5 |
| | Squalane | 5.0 | 5.0 | 5.0 | 5.0 | 5.0 | 5.0 | 5.0 |
| | Cetyl 2-ethylhexanoate | 5.0 | 5.0 | 5.0 | 5.0 | 5.0 | 5.0 | 5.0 |
| | Methylpolysiloxane | 0.5 | 0.5 | 0.5 | 0.5 | 0.5 | 0.5 | 0.5 |
| | Propylparaben | 0.05 | 0.05 | 0.05 | 0.05 | 0.05 | 0.05 | 0.05 |
| | Methylparaben | 0.1 | 0.1 | 0.1 | 0.1 | 0.1 | 0.1 | 0.1 |
| Liquid B | L-carnitine 2-hexyldecanoate hydrochloride | 1.0 | 5.0 | | | | | |
| | L-carnitine 2-methylpalmitate hydrochloride | | | 1.0 | 5.0 | | | |
| | L-carnitine 2-butyloctanoate hydrochloride | | | | | 1.0 | 5.0 | |
| | 1,3-Butylene glycol | 7.0 | 7.0 | 7.0 | 7.0 | 7.0 | 7.0 | 7.0 |
| | NaOH | 0.1 | 0.5 | 0.1 | 0.5 | 0.1 | 0.5 | |
| | Purified water | 9.0 | 9.0 | 9.0 | 9.0 | 9.0 | 9.0 | 9.0 |
| Liquid C | Xanthan gum | 0.1 | 0.1 | 0.1 | 0.1 | 0.1 | 0.1 | 0.1 |
| | Purified water | Balance | Balance | Balance | Balance | Balance | Balance | Balance |

| | | | | | | | | |
|---|---|---|---|---|---|---|---|---|
| (The values are % by weight relative to the total weight at 100.) | | | | | | | | |

### [Formulation Examples 8 to 11]

Cosmetics were prepared according to the compositions shown in Table 2 below. Glyceryl fatty acid esters and polyglyceryl fatty acid esters were used as nonionic surfactants.

Ingredients shown in Table 2 below were blended and were homogeneously suspended and dissolved while heating to 85°C to give liquids A, B and C. While the liquid A was stirred with a homomixer, the liquid B and the liquid C were gradually added thereto in this order. The mixtures were continuously stirred while being ice-cooled to approximately 30°C, and were thereafter naturally cooled to room temperature to give cosmetics 8 to 11.

### [Table 2]

**Table 2**

| | Ingredients | Amounts (%) | | | |
|---|---|---|---|---|---|
| | | Cosmetic 8 | Cosmetic 9 | Cosmetic 10 | Cosmetic 11 |
| Liquid A | Polyglyceryl-10 monostearate | 2.0 | 2.0 | 2.0 | 2.0 |
| | Glyceryl stearate | 1.0 | 1.0 | 1.0 | 1.0 |
| | Hydrogenated rapeseed oil alcohol | 4.2 | 4.2 | 4.2 | 4.2 |
| | Isononyl isononanoate | 6.0 | 6.0 | 6.0 | 6.0 |
| | Squalane | 10.0 | 10.0 | 10.0 | 10.0 |
| | Octyldodecyl myristate | 4.5 | 4.5 | 4.5 | 4.5 |
| | Xanthan gum | 0.1 | 0.1 | 0.1 | 0.1 |
| Liquid B | 1,2-Hexanediol | 5.0 | 5.0 | 5.0 | 5.0 |
| | L-carnitine 2-hexyldecanoate hydrochloride | 1.0 | | | |
| | L-carnitine 2-methylpalmitate hydrochloride | | 1.0 | | |
| | L-carnitine 2-butyloctanoate hydrochloride | | | 1.0 | |
| | KOH | 0.1 | 0.1 | 0.12 | |
| | Water | 10.0 | 10.0 | 10.0 | 10.0 |
| Liquid C | Glycerin | 4.8 | 4.8 | 4.8 | 4.8 |
| | Water | Balance | Balance | Balance | Balance |

| | | | | | |
|---|---|---|---|---|---|
| (The values are % by weight relative to the total weight at 100.) | | | | | |

### [Comparative Examples]

Cosmetics containing no nonionic surfactants were prepared according to the compositions shown in Table 3 below.

Ingredients shown in Table 3 below were blended and were homogeneously suspended and dissolved while heating to 85°C to give liquids A, B and C. While the liquid A was stirred with a homomixer, the liquid B and the liquid C were gradually added thereto in this order. The mixtures were continuously stirred while being ice-cooled to approximately 30°C, and were thereafter naturally cooled to room temperature to give cosmetics 12 to 15.

### [Table 3]

**Table 3**

| | Ingredients | Amounts (%) | | | |
|---|---|---|---|---|---|
| | | Cosmetic 12 | Cosmetic 13 | Cosmetic 14 | Cosmetic 15 |
| Liquid A | Stearic acid | 3.2 | 3.2 | 3.2 | 3.2 |
| | Behenyl alcohol | 3.2 | 3.2 | 3.2 | 3.2 |
| | Isopropyl palmitate | 4.8 | 4.8 | 4.8 | 4.8 |
| | Squalane | 6.4 | 6.4 | 6.4 | 6.4 |
| | 2-Octyl dodecanol | 4.8 | 4.8 | 4.8 | 4.8 |
| | Dimethicone | 0.4 | 0.4 | 0.4 | 0.4 |
| | Propylparaben | 0.05 | 0.05 | 0.05 | 0.05 |
| Liquid B | 1,2-Hexanediol | 4.8 | 4.8 | 4.8 | 4.8 |
| | L-carnitine 2-hexyldecanoate hydrochloride | 1.0 | | | |
| | L-carnitine 2-methylpalmitate hydrochloride | | 1.0 | | |
| | L-carnitine 2-butyloctanoate hydrochloride | | | 1.0 | |
| | Purified water | 10.0 | 10.0 | 10.0 | 10.0 |
| Liquid C | Concentrated glycerin | 4.8 | 4.8 | 4.8 | 4.8 |
| | 10% KOH | 2.6 | 2.6 | 3.0 | 1.6 |
| | Purified water | Balance | Balance | Balance | Balance |

| | | | | | |
|---|---|---|---|---|---|
| (The values are % by weight relative to the total weight at 100.) | | | | | |

### [Test Example 1]

The cosmetics 1 to 15 prepared in Formulation Examples 1 to 11 and Comparative Examples were each placed in a 50 ml wide-mouth glass bottle. The bottles were tightly closed and were allowed to stand at 25°C. The emulsion state was visually observed after predetermined time periods. The judge results are set forth in Table 4. The cosmetics containing the carnitine derivatives, in particular at a high concentration, showed a tendency to be unstable in emulsion state. In contrast, the cosmetics that contained the nonionic surfactants tended to maintain a stable emulsion state over longer periods compared to the cosmetics without the nonionic surfactants.

### [Table 4]

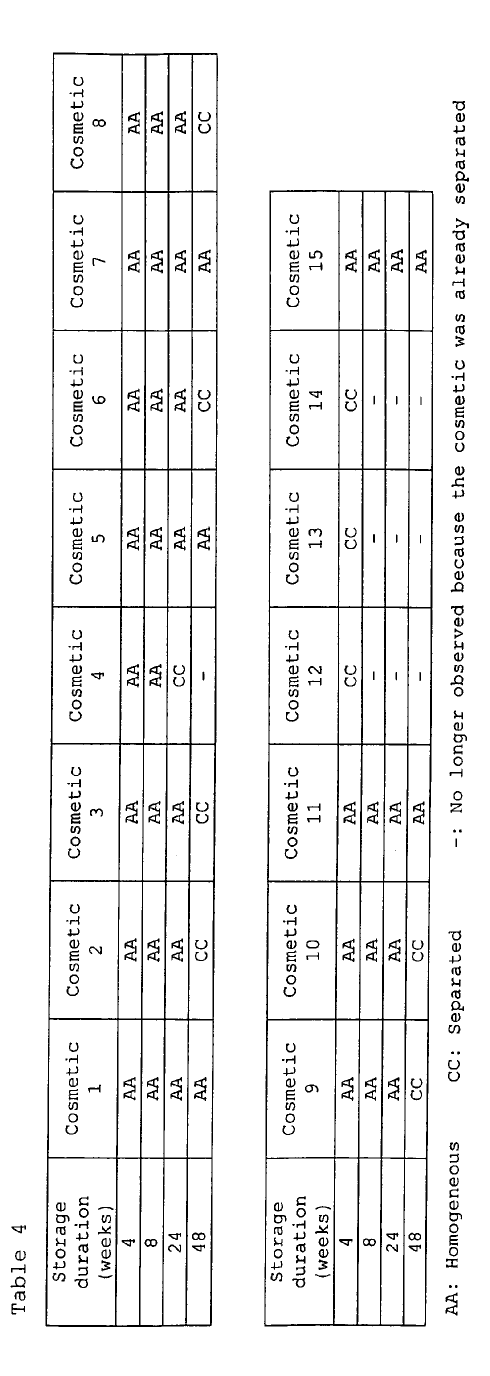

### [Test Example 2] pH dependency of formulation stability

Cosmetics having a pH in the range of 4.5 to 10 were prepared according to the formulation for the cosmetic 1 in the above Table 1 while adjusting the amount of NaOH (0.1% by weight in cosmetic 1). For reference, the pH of the cosmetic 1 was 7.0.

These cosmetics were placed in respective 50 ml wide-mouth glass bottles, and the bottles were tightly closed and allowed to stand at 25°C. The emulsion state of the cosmetics was visually observed after predetermined time periods. The judge results are set forth in Table 5.

### [Table 5]

**Table 5**

| pH of cosmetics | Cosmetic state | | | |
|---|---|---|---|---|
| | 8 weeks | 24 weeks | 48 weeks | 72 weeks |
| 4.5 | CC | - | - | - |
| 5 | AA | AA | AA | CC |
| 5.5 | AA | AA | AA | AA |
| 6 | AA | AA | AA | AA |
| 6.5 | AA | AA | AA | AA |
| 7 (cosmetic 1) | AA | AA | AA | AA |
| 7.5 | AA | AA | AA | AA |
| 8 | AA | AA | AA | AA |
| 8.5 | AA | AA | AA | AA |
| 9 | AA | AA | AA | CC |
| 9.5 | AA | AA | CC | - |
| 10 | CC | - | - | - |

| | | | | |
|---|---|---|---|---|
| AA: Homogeneous CC: Separated -: No longer observed because the cosmetic was already separated | | | | |

### [Test Example 3] pH dependency of formulation stability

Cosmetics having a pH in the range of 4.5 to 10 were prepared according to the formulation for the cosmetic 8 in the above Table 2 while adjusting the amount of KOH (0.1% by weight in cosmetic 8). For reference, the pH of the cosmetic 8 was 7.0.

These cosmetics were placed in respective 50 ml wide-mouth glass bottles, and the bottles were tightly closed and allowed to stand at 25°C. The emulsion state of the cosmetics was visually observed after predetermined time periods. The judge results are set forth in Table 6.

### [Table 6]

**Table 6**

| pH of cosmetics | Cosmetic state | | | |
|---|---|---|---|---|
| | 4 weeks | 8 weeks | 12 weeks | 24 weeks |
| 4.5 | CC | - | - | - |
| 5 | AA | AA | CC | - |
| 5.5 | AA | AA | AA | AA |
| 6 | AA | AA | AA | AA |
| 6.5 | AA | AA | AA | AA |
| 7 (cosmetic 8) | AA | AA | AA | AA |
| 7.5 | AA | AA | AA | AA |
| 8 | AA | AA | AA | AA |
| 8.5 | AA | AA | AA | AA |
| 9 | AA | AA | AA | CC |
| 9.5 | AA | CC | - | - |
| 10 | CC | - | - | - |

| | | | | |
|---|---|---|---|---|
| AA: Homogeneous CC: Separated -: No longer observed because the cosmetic was already separated | | | | |

## Claims

1. An emulsified skin external preparation comprising 0.01 to 10% by mass of a carnitine derivative represented by Formula (1) and/or a carnitine derivative salt represented by Formula (2), and 0.01 to 20% by mass of a nonionic surfactant; 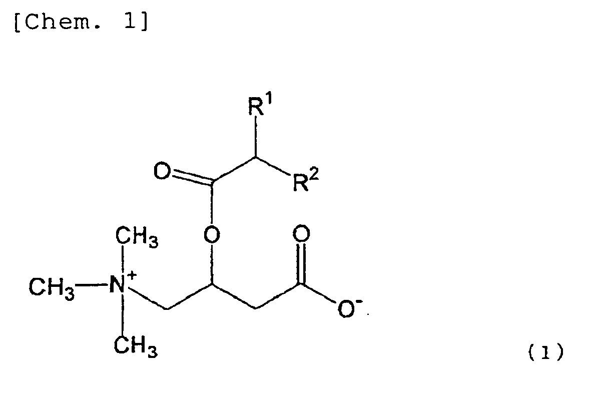 wherein R¹ and R² are each independently a C1-18 optionally branched, saturated or unsaturated aliphatic hydrocarbon group; 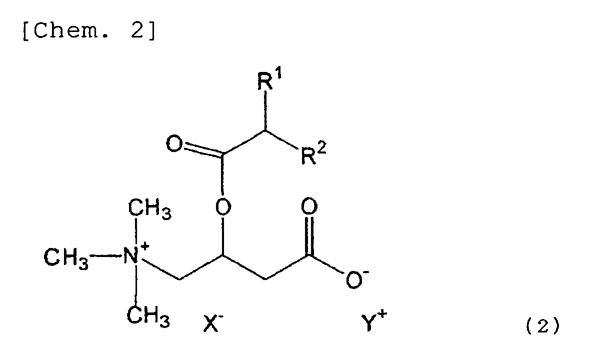 wherein R¹ and R² are the same as R¹ and R² in Formula (1), X⁻ is an inorganic or organic anion providing electrical neutrality with the cation part of the carnitine derivative, and Y⁺ is an inorganic or organic cation providing electrical neutrality with the anion part of the carnitine derivative.

2. The emulsified skin external preparation according to claim 1, wherein R¹ and R² in Formulae (1) and (2) are each independently a C3-16 optionally branched, saturated or unsaturated aliphatic hydrocarbon group.

3. The emulsified skin external preparation according to claim 1, wherein either of R¹ and R² in Formulae (1) and (2) is a C6 linear alkyl group and the other is a C8 linear alkyl group.

4. The emulsified skin external preparation according to any one of claims 1 to 3, wherein X⁻ in Formula (2) is selected from the group consisting of hydroxide ion, nitrate ion, sulfate ion, carbonate ion, hydrogen carbonate ion, halide ion, formate ion, acetate ion, citrate ion, tartrate ion, oxalate ion, fumarate ion, C3-20 optionally branched, saturated or unsaturated fatty acid anion, carnitine anion, carnitine derivative anion, ascorbate anion, ascorbylphosphate anion and ascorbylphosphate derivative anion.

5. The emulsified skin external preparation according to any one of claims 1 to 4, wherein Y⁺ in Formula (2) is selected from the group consisting of hydrogen ion, sodium ion, potassium ion, calcium ion, magnesium ion, zinc ion, ammonium ion, carnitine cation and carnitine derivative cation.

6. The emulsified skin external preparation according to any one of claims 1 to 5, wherein the nonionic surfactant has a polyoxyethylene skeleton.

7. The emulsified skin external preparation according to claim 6, wherein the nonionic surfactant is a nonionic ether ester surfactant.

8. The emulsified skin external preparation according to claim 7, wherein the nonionic surfactant is a polyoxyethylene polyhydric alcohol fatty acid ester.

9. The emulsified skin external preparation according to any one of claims 1 to 8, wherein the nonionic surfactant is a polyoxyethylene sorbitan fatty acid ester.

10. The skin external preparation according to any one of claims 1 to 9, wherein the preparation has a pH in the range of 5.0 to 9.5.

11. The skin external preparation according to any one of claims 1 to 9, wherein the preparation has a pH in the range of 5.5 to 8.5.

12. The emulsified skin external preparation according to any one of claims 1 to 11, which is used as a cosmetic.
